(19) Europäisches Patentamt
European Patent Office
Office européen des brevets

(11) **EP 2 913 073 B1**

(12) **EUROPÄISCHE PATENTSCHRIFT**

(45) Veröffentlichungstag und Bekanntmachung des Hinweises auf die Patenterteilung:
**10.04.2019 Patentblatt 2019/15**

(51) Int Cl.:
***A61M 1/36*** *(2006.01)*

(21) Anmeldenummer: **15154748.6**

(22) Anmeldetag: **11.02.2015**

(54) **Vorrichtung zur Erkennung einer venösen Nadeldiskonnektion**

Device for detecting a venous needle disconnection

Dispositif destinés à la détection d'une déconnexion d'aiguille veineuse

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.02.2014 DE 102014102731**

(43) Veröffentlichungstag der Anmeldung:
**02.09.2015 Patentblatt 2015/36**

(73) Patentinhaber: **B. Braun Avitum AG**
**34212 Melsungen (DE)**

(72) Erfinder:
• **Wolff, Henrik**
**37213 Witzenhausen (DE)**
• **Ritter, Kai-Uwe**
**91126 Rednitzhembach (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(56) Entgegenhaltungen:
**EP-A1- 2 218 470   DE-A1-102008 059 379**
**US-A1- 2003 128 125   US-A1- 2008 065 006**
**US-A1- 2010 022 934**

Bemerkungen:
Die Akte enthält technische Angaben, die nach dem Eingang der Anmeldung eingereicht wurden und die nicht in dieser Patentschrift enthalten sind.

EP 2 913 073 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zur Erkennung einer venösen Nadeldiskonnektion sowie eine hiermit ausgestattete Vorrichtung zur extrakorporalen Blutbehandlung. Die Vorrichtung zur extrakorporalen Blutbehandlung kann zur Durchführung einer Blutbehandlungstherapie wie z.B. einer Hämodialyse, Hämofiltration oder einer Hämodiafiltration ausgelegt sein. Hierbei wird vorzugsweise eine Vorrichtung zur Überwachung eines Gefäßzugangs während einer extrakorporalen Blutbehandlung eingesetzt.

Zur Blutbehandlung kann das Blut eines Patienten beispielsweise im Zuge einer Hämodialyse, Hämofiltration oder Hämodiafiltration über einen extrakorporalen Blutkreislauf geleitet werden. Um Zugang zum Blutgefäßsystem des Patienten zu erhalten, können arteriovenöse Fisteln, Shunts oder auch Gefäßimplantate verwendet werden. Die Verbindung des extrakorporalen Blutkreislaufs mit dem Patienten erfolgt üblicherweise über Katheter oder Kanülen bzw. Nadeln, z.B. Dialysekanülen oder - nadeln, mit denen beispielsweise eine Fistel oder ein Shunt bzw. ein Gefäßimplantat punktiert wird und hierdurch eine Fluidverbindung hergestellt wird.

Bei Beginn oder auch während einer Blutbehandlung kann der Fall auftreten, dass der venöse Zugang zum Blutkreislauf gestört wird, wenn beispielsweise die Nadel oder Kanüle verrutscht und der extrakorporale Kreislauf nicht mehr ordnungsgemäß, oder überhaupt nicht mehr mit dem intrakorporalen Blutkreislauf, auch Patientenblutkreislauf, verbunden ist. Dies kann insbesondere bei einer Diskonnektion des venösen Zugangs zum Patientenblutkreis problematisch werden. Wird eine solche Diskonnektion des venösen Zugangs nicht rechtzeitig erkannt, wird über den arteriellen Zugang weiterhin Blut aus dem Patienten entnommen, aber nicht mehr ordnungsgemäß in den Patientenkörper nach der extrakorporalen Blutbehandlung zurückgeleitet. Bei üblichen Blutflussraten von beispielsweise 300 bis 400 ml/min entwickelt sich innerhalb weniger Minuten eine lebensgefährliche Situation.

In der EP 1 584 339 B1 ist ein Verfahren zum Erkennen einer Nadeldiskonnektion auf der Basis der Messung von arteriellen und venösen Drücken unter Summen- und Differenzbildung offenbart.

Bei einer Vorrichtung gemäß US 7,648,474 B2 werden die arteriellen und venösen Druckwerte zum Ermitteln einer Nadeldiskonnektion überwacht.

EP 2 218 470 A1 zeigt ein Hämodialyse-Gerät, bei dem medizinische Störfälle wie etwa ein Blutverlust oder ein Lufteinschluss während einer medizinischen Behandlung vermieden werden sollen, in dem Verbindungsfehler zwischen dem Blutkreislauf und dem Hämodialyse-Gerät erkannt werden können. Wenn eine vorbestimmte Zeitspanne ab dem Beginn eines Kontrollzyklus verstrichen ist, kann eine Blutklemme geschlossen und danach nach Verstreichen eines vorbestimmten Zeitintervalls wieder geöffnet werden, wobei eine Beurteilungseinrichtung eine Druckveränderung in dem Dialysatdruck oder dem venösen Druck für eine Zeitspanne vergleicht, wenn die Klemme geschlossen ist, und zwar mit einem vorbestimmten Referenzwert, um eine Normalität der Schaltungsverbindung zu beurteilen.

Druckschrift US 2003/0128125 A1 offenbart eine Vorrichtung und ein entsprechendes Verfahren, bei dem mehrere Sensoren mit Alarmbedingungsdetektoren verbunden sind, denen ein Alarmzustandsklassifizierer sowie eine Alarmsteuereinrichtung mit entsprechenden Ausgängen nachgeschaltet sind. Damit soll das Problem von Falschindikationen verringert werden.

Druckschrift US 2008/065006 A1 betrifft eine verbesserte Signaldetektion für Systeme zur Erfassung einer Zugangs-Diskonnektion, bei der ein extrakorporales System mit dem Patienten über eine Blutströmungserfassung sowohl im Zustrom als auch in der Rückführung versehen ist.

Mit der Erfindung wird eine Vorrichtung gemäß Anspruch 1 bereitgestellt.

Mit der erfindungsgemäßen Vorrichtung lässt sich beispielsweise eine venöse Nadeldiskonnektion frühzeitig erkennen und hierdurch hohe Patientensicherheit bei einer Behandlung wie etwa einer Dialysebehandlung, und auch eine hohe Funktionssicherheit der erfindungsgemäßen Vorrichtung gewährleisten.

Weiterbildungen der Erfindung sind in den abhängigen Ansprüchen definiert.

Ausführungsbeispiele der erfindungsgemäßen Vorrichtung dienen zur Erkennung einer Verbindungsunterbrechung zwischen einer Blutbehandlungsvorrichtung und einem Patientenblutkreislauf, der mit der Blutbehandlungsvorrichtung über eine Verbindungseinrichtung, beispielsweise eine Nadel, in Verbindung bringbar ist. Die Verbindungseinrichtung, beispielsweise die Nadel, ist an einer Fluidleitung, optional an deren Ende, anbringbar. Ein oder mehrere Ausführungsbeispiele der erfindungsgemäßen Vorrichtung weisen eine Leitungsabsperrung, einen Drucksensor, der dazu ausgelegt ist, den in der Fluidleitung herrschenden Fluiddruck an einer Position zu messen, die zwischen der Leitungsabsperrung und der Verbindungseinrichtung liegt, und eine Steuer- und Auswerteeinrichtung auf, die dazu ausgelegt ist, den nach Absperrung der Leitung auftretenden Druck oder Druckverlauf auszuwerten, um hieraus eine Verbindungsunterbrechung oder Verbindungsstörung zwischen der Verbindungseinrichtung und dem Patientenblutkreislauf zu erkennen. Eine Verbindungsunterbrechung oder Verbindungsstörung wird vorstehend und nachstehend auch als Nadeldiskonnektion bezeichnet.

Die Vorrichtung kann Bestandteil einer Blutbehandlungsvorrichtung, beispielsweise einer Dialysevorrichtung sein. Eine Anzeige- und/oder Alarmeinrichtung kann zur Erzeugung einer Anzeige oder eines Alarms im Falle der Detektion einer Verbindungsunterbrechung oder Verbindungsstörung vorgesehen sein.

Die Vorrichtung kann dazu ausgelegt sein, den nach Schließen der Leitungsabsperrung kontinuierlich oder wiederholt gemessenen, in der Fluidleitung (31) auftretenden Druck mit einem Schwellwert zu vergleichen und/oder die Geschwindigkeit des Druckabfalls zu ermitteln und/oder einen sich tendenziell ergebenden Druckendwert zu ermitteln und/oder zu erfassen, ob der Druck gegen Atmosphärendruck oder einen höheren, im Patientenblutkreislauf auftretenden Druck tendiert.

Die Vorrichtung kann eine Blutpumpe aufweisen, die auch nach Betätigung der Leitungsabsperrung mit entsprechender Fluidsperrung des Fluidflusses durch die Leitungsabsperrung hindurch weiter betreibbar ist, wobei das geförderte Fluid in einer Sammeleinrichtung, beispielsweise einem Blasenfänger, zwischenspeicherbar ist.

Die Vorrichtung kann dazu ausgelegt sein, den Betrieb der Blutpumpe zu beenden, wenn eine das geförderte Fluid aufnehmende Sammeleinrichtung bei geschlossener Leitungsabsperrung gefüllt ist und/oder wenn eine Diskonnektion der Verbindungseinrichtung ermittelt wird und/oder wenn ein durch den Drucksensor gemessener Fluiddruck einen bestimmten Grenzwert erreicht oder unterschreitet.

Der Patientenblutkreislauf kann bei der Vorrichtung über eine Verbindungseinrichtung, beispielsweise eine Nadel, mit der Blutbehandlungsvorrichtung in Verbindung stehen. Die Verbindungseinrichtung, beispielsweise die Nadel, kann an einer Fluidleitung, optional an deren Ende, angebracht sein. Eine oder mehrere der Ausführungsformen der Vorrichtung können dazu ausgelegt sein, über einen Drucksensor der in der Fluidleitung herrschende Fluiddruck in regelmäßigen oder unregelmäßigen Zeitabständen an einer Position zu messen, die zwischen einer Leitungsabsperrung und der Verbindungseinrichtung liegt, wobei die Fluidleitung in regelmäßigen oder unregelmäßigen Zeitabständen abgesperrt werden kann und der in der Fluidleitung nach Absperrung der Fluidleitung auftretende Druck oder Druckverlauf ausgewertet werden kann, um hieraus eine Verbindungsunterbrechung oder Verbindungsstörung zwischen der Verbindungseinrichtung und dem Patientenblutkreislauf zu erkennen.

Im Falle der Detektion einer Verbindungsunterbrechung kann eine Anzeige oder ein Alarm erzeugt werden.

Der bei diesen Ausführungsformen der Vorrichtung nach Schließen der Leitungsabsperrung kontinuierlich oder wiederholt gemessene, in der Fluidleitung auftretende Druck kann mit einem Schwellenwert verglichen werden und/oder es kann die Geschwindigkeit des Druckabfalls ermittelt werden und/oder ein zu erwartender Druckendwert ermittelt werden, und/oder erfasst werden, ob der Druck gegen Atmosphärendruck oder einen höheren, im Patientenblutkreislauf auftretenden Druck tendiert.

Die Vorrichtung kann eine Blutpumpe kann auch nach Betätigung der Leitungsabsperrung mit entsprechender Fluidsperrung des Fluidflusses durch die Leitungsabsperrung hindurch weiter betrieben werden, wobei das geförderte Fluid in einer Sammeleinrichtung, beispielsweise einem Blasenfänger, zwischenspeicherbar ist.

Die Vorrichtung kann dazu ausgelegt sein, den Betrieb der Blutpumpe zu beenden, wenn ein das geförderte Fluid aufnehmender Sammelbehälter bei geschlossener Leitungsabsperrung gefüllt ist, und/oder wenn eine Diskonnektion der Verbindungseinrichtung ermittelt wird, und/oder wenn ein durch den Drucksensor gemessener Fluiddruck einen bestimmten Grenzwert erreicht oder unterschreitet.

Die Vorrichtung kann dazu ausgelegt sein, die Leitungsabsperrung wieder zu öffnen, sobald erkannt wird, dass keine Verbindungsunterbrechung oder Verbindungsstörung zwischen der Blutbehandlungsvorrichtung und der Verbindungseinrichtung vorliegt.

Die Erfindung wird nachstehend unter Bezugnahme auf die Zeichnungen und Ausführungsbeispiele näher erläutert. Es zeigen:

Fig. 1 ein Ausführungsbeispiel einer erfindungsgemäßen Vorrichtung;

Fig. 2 einen Druckverlauf vor und nach einer Nadeldiskonnektion;

Fig. 3 eine Ausführungsform eines nicht beanspruchten Verfahrens zum Erkennen einer Nadeldiskonnektion;

Fig. 4 ein Diagramm des zeitlichen Druckverlaufs bei einer Nadeldiskonnektion und einer nachfolgenden Diskonnektionsüberprüfung;

Fig. 5 ein Beispiel eines Druckverlaufs sowie der zeitlichen Abfolge der Schritte einer nicht beanspruchten Ausführungsform;

Fig. 6 in zwei Kurvenzügen zeitliche Druckverläufe vor, während und nach einer Fluidströmungsabsperrung;

Fig. 7 illustriert zwei Beispiele der Druckverläufe bei ordnungsgemäßer Nadelkonnektion bzw. bei einer Nadeldiskonnektion; und

Fig. 8 zwei exemplarische Druckverläufe in ihrem zeitlichen Verlauf bei einer Konnektion oder Diskonnektion.

Mit Ausführungsbeispielen der erfindungsgemäßen Vorrichtung ist es möglich, sogenannte venöse Nadeldiskonnektionen oder Verlagerungen (VND, "Venous Needle Dislodgement") zu erkennen. Beispielsweise kann aufgrund von Bewegungen des Patienten oder einer unzureichenden Befestigung der Nadel ein Verrutschen bis hin zum gänzlichen Herausrutschen der Nadel aus der Einstichposition auftreten. Dies ist insbesondere im Bereich der venösen Nadel problematisch, da über diese das Blut zu dem Patienten zurückgeführt wird, wobei das Blut hier unter entsprechendem Druck steht. Dies kann die Gefahr einer unerwünschten Verlagerung bis hin zu einem Herausrutschen der Nadel noch weiter erhöhen.

Im vorliegenden Zusammenhang ist unter dem Ausdruck Nadeldiskonnektion sowohl ein vollständiges Herausrutschen der Nadel aus dem venösen Gefäßzugang als auch ein nur teilweises Dislozieren der Nadel mit eingeschränkter, aber noch in gewissem Umfang vorhandener Verbindung zum Blutkreislauf zu verstehen. Bei nur partiellem Verrutschen der Nadel kann die Nadel in dem die eigentliche Zugangsstelle umgebenden Gewebe liegen und in diesem eine paravasale Blutung hervorrufen.

Zur Erkennung einer venösen Nadeldiskonnektion kann allgemein der venöse Druck überwacht werden. Allerdings kann vor allem bei kleineren Kanülenquerschnitten der Druckabfall an der Kanüle recht hoch sein, sodass selbst bei erfolgter venöser Nadeldiskonnektion kein signifikanter Druckabfall erkennbar ist. Auch hydrostatische Druckschwankungen können die Erkennung von dislozierungsbedingten Druckschwankungen erschweren oder unmöglich machen.

Bei Ausführungsbeispielen der erfindungsgemäßen Vorrichtung ist vorgesehen, den Fluidfluss, also den Blutfluss, vor oder während der Durchführung einer Druckmessung oder Drucküberwachung anzuhalten oder zumindest einzuschränken. Hierdurch lässt sich das vorstehend genannte Problem eines hohen Flusswiderstands in der Kanüle mit dem damit verbundenen Offset im Messsignal zumindest reduzieren.

Im Folgenden wird als extrakorporaler Kreislauf eine Blutführung, z.B. ein System oder eine Anordnung bezeichnet, bei der ein einem Gegenstand oder einem Patienten entnommenes Fluid, z.B. Blut, außerhalb des Patientenblutkreislaufs in dem extrakorporalen Kreislauf geführt und anschließend dem Patientenblutkreislauf wieder zugeführt wird. Der venöse Zweig des extrakorporalen Kreislaufs kann beispielsweise einen das Fluid von der Behandlungseinrichtung zum Gegenstand oder Patienten zurückführenden, also das Fluid abführenden Schlauch, der auch als Blutabführleitung des Dialysators ausgebildet sein kann, umfassen.

Ausführungsbeispiele der Erfindung ermöglichen nicht nur die Erkennung einer ungenügenden Verbindung einer Kanüle wie etwa einer venösen Kanüle bis hin zum Herausrutschen der Kanüle aus dem vorgesehenen Zugangsort, sondern auch das Erfassen z.B. eines Blutlecks im venösen Zweig hinter der Vorrichtung zur Erkennung einer Verbindungsunterbrechung. Dies kann beispielsweise bei einem Zwei-Nadel-Betrieb der Blutbehandlungsmaschine wie etwa der Dialysemaschine realisiert sein.

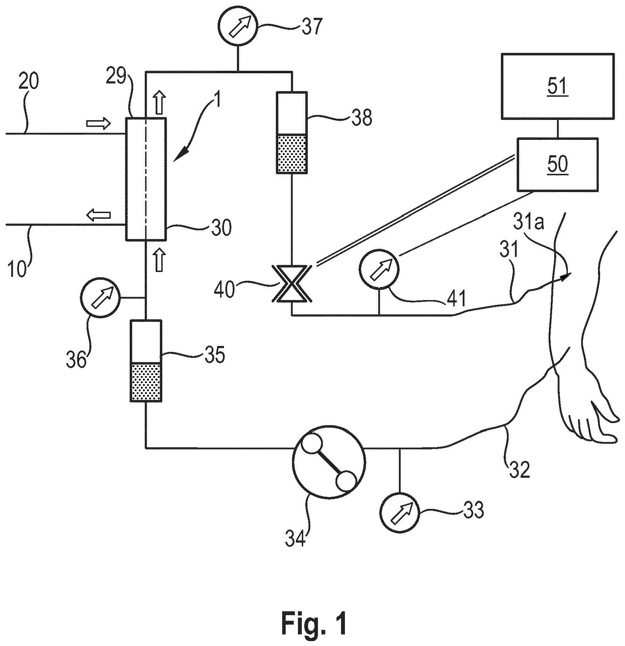

Fig. 1 zeigt ein Ausführungsbeispiel einer erfindungsgemäßen Dialysevorrichtung sowie ein Ausführungsbeispiel einer erfindungsgemäßen Vorrichtung zur Erkennung einer venösen Nadeldiskonnektion sowie einer Ausführungsform zur Erkennung einer solchen venösen Nadeldiskonnektion.

In Fig. 1 ist ein Ausführungsbeispiel einer Dialysevorrichtung gezeigt, die mit einem Ausführungsbeispiel eines Systems zur Erkennung einer venösen Nadeldiskonnektion ausgestattet ist. Das in Fig. 1 dargestellte Ausführungsbeispiel der erfindungsgemäßen Vorrichtung zur Erkennung einer fehlerhaften oder fehlenden Verbindung einer venösen Verbindung zwischen dem extrakorporalen Blutkreislauf und dem Patientenblutkreislauf ist anhand einer Dialysemaschine erläutert. Anstelle oder zusätzlich zu einer Hämodialyse kann aber auch eine reine Hämofiltration oder eine Hämodiafiltration realisiert sein.

Das Ausführungsbeispiel gemäß Fig. 1 umfasst einen Dialysator 1, der durch eine semipermeable Membran in eine erste, in einem Dialysierflüssigkeitsweg angeordnete Kammer 29 und eine zweite Kammer 30 unterteilt ist, die ihrerseits mittels einer vom Patientenblutkreislauf abführenden Blutzuführleitung 32 mit dem Dialysatoreingang verbunden ist. Die zweite Kammer 30 ist mittels eines Dialysatorausganges mit einer zum Patienten führenden Blutabführleitung 31 mit dem Blutkreislauf des Patienten verbindbar, um diesem das gereinigte Blut wieder zuzuführen. Die erste Kammer 29 ist mit einem Zulauf 20 für frische Dialysierflüssigkeit und einem Ablauf 10 für verbrauchte Dialysierflüssigkeit verbunden.

Über einen Drucksensor 41 kann der venöse Druck nach Abklemmen einer Schlauchsperre (Schlauchabsperrklemme oder Schlauchklemme) 40 und/oder nach Anhalten einer Blutpumpe 34 erfasst werden. Die Schlauchklemme 40 kann manuell oder automatisch betätigt werden und eine vollständige oder auch nur partielle Sperrung des Fluidströmungskreislaufs zurück zum venösen Zugang des Patienten bewirken. Bei dem Ausführungsbeispiel gemäß Fig. 1 ist der zusätzliche Drucksensor 41 für den venösen Druck zwischen der Schlauchklemme 40 und dem venösen Patientenzugang angeordnet und liegt damit im Blutrücklauf zum Patienten. Da der Drucksensor 41 an der Blutabführleitung 31 unmittelbar vor dem Patienten oder jedenfalls stromab der Schlauchklemme 40 angeordnet ist, stellt sich am Drucksensor 41 bei ordnungsgemäßer Verbindung mit dem Patientenblutkreislauf der Druck aus dem Patientenzugang ein, wohingegen bei ganz oder teilweise aus dem Patientenzugang herausgetretener Nadel der Atmosphärendruck gemessen wird.

Das Ausführungsbeispiel weist den Drucksensor 41 in dem Blut zu einer venösen Nadel 31a zuführenden Schlauch (Blutabführleitung) 31 auf, der nach der Schlauchabsperrung bzw. Schlauchklemme 40 und vor der Nadel 31a, d.h. an dem Leitungsabschnitt zwischen der Schlauchabsperrung 40 und der Nadel 31a, angeordnet ist. Wie in Fig. 1 gezeigt ist, sind im arteriellen Zweig 32 des extrakorporalen Blutkreislaufs, d.h. in dem von dem Patienten zum Dialysatoreingang führenden Kreislauf, ein Drucksensor 33 an der vom Patienten zum Dialysatoreingang führenden Blutzuführleitung 32 und ein Blasenfänger 35 angeordnet, zwischen denen die Blutpumpe 34 in die Blutzuführleitung 32 eingefügt ist. Ein weiterer Drucksensor 36 erfasst den Druck des Bluts unmittelbar am Dialysatoreingang des Dialysators 1. Die Dialysierflüssigkeit wird in der durch die Pfeile symbolisierten Richtung über die Dialysierflüssigkeitszuleitung 20 zu einer ersten Kammer 29 des Dialysators 1 geleitet und aus dieser über die Dialysierflüssigkeitsabführleitung 10 zum Auslass oder Abfall geleitet. Über die zweite Kammer 30 des Dialysators 1 strömt das zu reinigende Blut und wird am Dialysatorausgang in Pfeilrichtung zur Blutabführleitung 31 geleitet.

In der Blutabführleitung 31 ist ein weiterer Blasenfänger 38 optional vorgesehen. Ein Drucksensor 37 erfasst den Druck in der Blutabführleitung 31 am Ausgang des Dialysators 1.

Die Strömungsabsperrung 40, die auch als Schlauchabsperrung oder als Schlauchabsperrklemme ausgeführt sein kann, befindet sich in Blutströmungsrichtung gesehen stromauf des Drucksensors 41, der an oder in der Blutabführleitung 31 angeordnet ist. Die Blutzuführleitung 32 und die Blutabführleitung 31 können beispielsweise als Schläuche ausgebildet sein.

Bei dem Ausführungsbeispiel wird das Blut eines Patienten in einem extrakorporalen Kreislauf geführt und fließt durch den Schlauch 32 in die blutseitige Kammer 30 des Dialysators 1, wonach das gereinigte Blut durch die Blutabführleitung 31 zum Patienten zurückgeführt wird.

Die Flussrate des Blutkreislaufs (extrakorporal) wird durch die Blutpumpe 34 kontrolliert, wobei am oder im Schlauch 32 stromauf (in Strömungsrichtung gesehen) die Drucksensoreinheit 33 für die arterielle Druckmessung angeordnet ist.

Der Blasenfänger 35 dient zum Abfangen der Luftblasen im arteriellen Schlauchsegment, wobei zwischen dem Blasenfänger 35 und dem Dialysator 1 eine weitere Drucksensoreinheit 36 vorgesehen ist.

Bei dem dargestellten Ausführungsbeispiel ist der Drucksensor 41 zwischen der Schlauchabsperrung 40 und dem Patienten an oder in der Blutabführleitung 31 angeordnet.

Über die Dialysierflüssigkeitszuführleitung 20 wird die Dialysierflüssigkeit der Dialysierflüssigkeitskammer 29 des Dialysators zugeführt, wobei die Dialysierflüssigkeitskammer 29 von der Blutkammer 30 durch eine semipermeable Membran getrennt ist. Die Dialysierflüssigkeit wird von einer nicht gezeigten Flüssigkeitsquelle bereitgestellt, fließt durch den Schlauch 20 und wird durch eine nicht gezeigte Pumpe in die Kammer 29 des Dialysators gepumpt, durchströmt diese und wird anschließend über das Schlauchsystem 10 einem Abfallbehälter zugeführt.

Im Blut zuführenden Schlauch (Blutzuführleitung) 31 ist nach dem Dialysator 1 der Drucksensor 37 für die venöse Druckmessung angeordnet, dem der Blasenfänger 38 nachfolgt. In Strömungsrichtung folgt hierauf die Schlauchabsperrung 40, beispielsweise in Form einer Schlauchabsperrklemme, um den Patienten venös abzuklemmen, beispielsweise im Fehlerfall oder gezielt zur Durchführung einer Erkennung einer Nadeldiskonnektion.

Wird die Blutpumpe 34 während der Messung auf venöse Nadeldiskonnektion nicht abgestellt, so ist vorzugsweise optional im Leitungsbereich zwischen der Blutpumpe 34 und der Schlauchabsperrung 40 ein Reservoir zur Aufnahme des geförderten Bluts vorgesehen. Diese Aufnahme des geförderten Bluts kann beispielsweise auch von den Blasenfängern 35 und/oder 38 übernommen werden.

Nach der Messung auf eventuelle Nadeldiskonnektion kann das im Blasenfänger 38 und/oder 35 zwischengespeicherte Blut nach Öffnung der Schlauchabsperrung 40 langsam wieder an den Patienten zurückgegeben werden, sodass das Speichervolumen der Blasenfängers 38 und/oder 35 bei einer nachfolgenden Messung wieder für eine Aufnahme von Blut zur Verfügung steht.

Bei einem oder mehreren Ausführungsbeispielen ist hierzu eine Pegelregulierung in den Speichervolumina der Blasenfänger 35 und/oder 38 vorgesehen, sodass der Pegel der in den Blasenfängern 35 und/oder 38 gespeicherten Fluidmenge bei geöffneter Schlauchabsperrung 40 auf einen gewünschten Pegel reguliert wird.

Eine Steuereinrichtung, z.B. in Form einer Datenverarbeitungs- und Speichereinheit 50 kontrolliert alle in Fig. 1 gezeigten Elemente über geeignete Schnittstellen, wobei in Fig. 1 nur die Verbindung mit dem Drucksensor 41 und der Schlauchabsperrung 40 dargestellt sind. Die Datenverarbeitungs- und Speichereinheit 50 sammelt Informationen auch über andere Parameter der Dialysevorrichtung, z.B. Blutfluss, Dialysierflüssigkeitsfluss und/oder Behandlungszeit. Diese Parameter werden zusammen mit den gemessenen Daten verarbeitet. Die Datenverarbeitungs- und Speichereinheit 50 dient zur Verarbeitung der gemessenen Druckwerte sowie zur Steuerung der Blutabsperrung und der Messzeitpunkte. Ferner kann eine interne oder externe Speichereinheit zur Speicherung der Messwerte sowie der Steuerungszeitverläufe vorhanden sein.

Die Messwerte der Drucksensoren 33, 36, 37 und 41 werden über Datenverbindungen, die kabelgebunden und/oder drahtlos ausgebildet sein können, zu der Datenverarbeitungs- und Speichereinheit 50 übertragen, die als Datenverarbeitungs- und Auswerteeinheit ausgebildet ist, um venöse Nadeldiskonnektionen zu erkennen.

Das Ausführungsbeispiel der erfindungsgemäßen Dialysemaschine oder der erfindungsgemäßen Vorrichtung zur Erkennung einer Nadeldiskonnektion weist weiterhin eine Ausgabeeinheit 51 in Form einer Warnsignaleinheit oder Anzeige auf, die über eine kabelgebundene oder drahtlose Datenverbindung mit der Datenverarbeitungs- und Speichereinheit 50 verbunden ist. Die Ausgabeeinheit 51 gibt ein optisches und/oder akustisches Warnsignal und/oder einen Text zur Erklärung eines Problemfalls beispielsweise auf einem Display an, wenn eine Fehlfunktion der Dialysemaschine und/oder der Vorrichtung zur Erkennung einer Nadeldiskonnektion, und/oder eine venöse Nadeldiskonnektion, erkannt werden. Des weiteren kann die Ausgabeeinheit 51 zur optischen, akustischen oder elektronischen Ausgabe oder einer Ausgabe in anderer Form, beispielsweise in Form elektronischer Speicherung, eines Ausdrucks, einer E-Mailübersendung oder dergleichen, ausgelegt sein.

Das Ausführungsbeispiel gemäß Fig. 1 kann weiterhin nicht dargestellte zusätzliche Messeinrichtungen, Pumpen, Blasenfänger usw. umfassen.

Bei Ausführungsbeispielen kann vorgesehen sein, die Blutpumpe 34 einfach anzuhalten oder zu drosseln, um die Blutströmung zu unterbinden oder zu reduzieren. Vorzugsweise erfolgt aber alternativ oder zusätzlich auch ein (vollständiges oder teilweises) Abklemmen des das zuzuführende, gereinigte Blut führenden Schlauchsystems vor der Messvorrichtung für die Detektion einer, z.B. venösen, Nadeldiskonnektion.

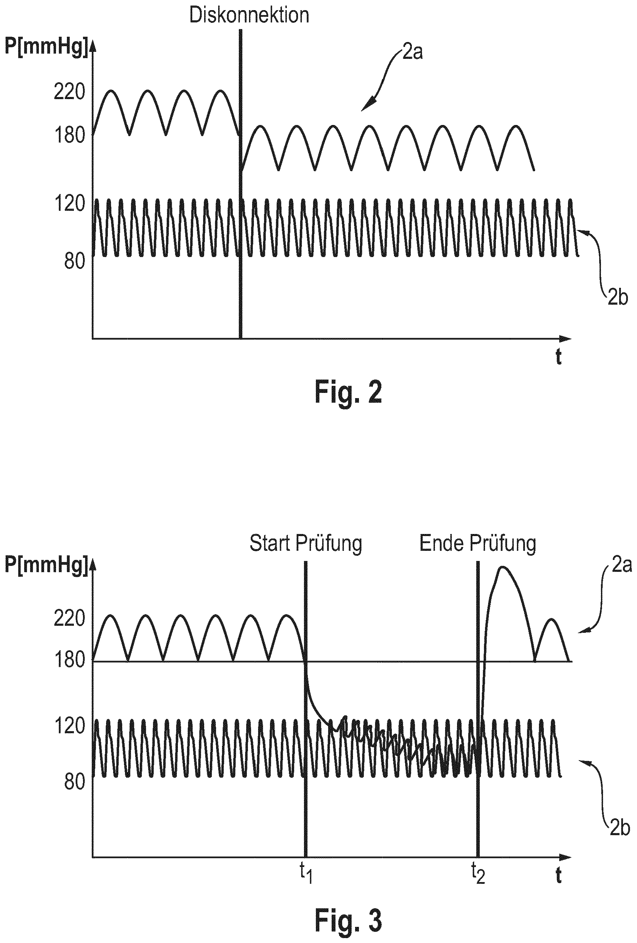

In Fig. 2 ist ein Beispiel eines Druckverlaufs am Drucksensor 37 für die venöse Druckmessung vor und nach einer venösen Nadeldiskonnektion, d.h. einer unerwünschten partiellen oder vollständigen Auftrennung der Verbindung zwischen der Dialysevorrichtung und dem Patientenblutkreislauf, dargestellt. Die obere Kurve 2a symbolisiert den venösen Druckverlauf, der sich in der Blutabführleitung 31 einstellt, während die untere, gleichförmige Kurve 2b den venösen Blutdruck im Zugang und damit im Körper des zu dialysierenden bzw. einer Blutbehandlung zu unterziehenden Patienten angibt. Die Wellenform des oberen Druckverlaufs 2a wird durch die Blutpumpe 34 erzeugt. Die untere Kurve 2b stellt den Shunt- bzw. Gefäßinnendruck dar und zeigt die durch den Herzschlag des Patienten aufgeprägte Pulsation. Der Zeitpunkt der unerwünschten Nadeldiskonnektion ist mit einer vertikalen Linie dargestellt. Wie ersichtlich, sinkt danach der venöse Druckverlauf um ca. 20 bis 40 mmHg ab. Aus Fig. 2 ist ersichtlich, dass der Druckabfall nach einer venösen Nadeldiskonnektion am Drucksensor 37 für die venöse Druckmessung relativ gering ist und lediglich ca. 20 bis 40 mmHg beträgt. Der Druckabfall liegt damit in einer Größenordnung, die auch durch andere Ursachen wie beispielsweise Bewegungen des Patienten oder Tieferstellen der Patientenliege hervorgerufen sein kann. Damit ist diese Absenkung nur recht schwierig zuverlässig zu detektieren.

Bei Ausführungsbeispielen der Erfindung ist die Unterscheidung der Ursache für die Druckänderung erheblich vereinfacht, da die durch die extrakorporale Blutpumpe 34 erzeugten Druckpulse nicht länger im Messsignal enthalten sind, wenn die extrakorporale Blutpumpe 34 abgeschaltet oder zumindest der Blutfluss gesperrt oder zumindest gedrosselt wird. Andernfalls liegen die Druckpulse nämlich ebenfalls in der Größenordnung der Druckänderungen.

Mit Ausführungsbeispielen der erfindungsgemäßen Vorrichtung, die auch als erfindungsgemäßes System bezeichnet werden kann, lassen sich venöse Nadeldiskonnektionen einfach, zuverlässig und rasch erkennen.

Bei dem Ausführungsbeispiel der erfindungsgemäßen Vorrichtung gemäß Fig. 1 dienen der Luftblasenfänger 35 und/oder 38 ggf. zudem als Speichervolumina, sodass die Blutpumpe 34 auch bei geschlossener Klemme 40 weiterlaufen kann. Alternativ oder zusätzlich kann die Blutpumpe 34 aber auch abgeschaltet oder hinsichtlich ihrer Förderrate bei geschlossener Klemme 40 reduziert weiter betrieben werden. Bei dem Ausführungsbeispiel erfolgt die Messung des Drucks durch den Drucksensor 41 bei gestopptem Blutfluss oder bei zumindest definiert verringertem Blutfluss. Bei diesem Ausführungsbeispiel der erfindungsgemäßen Vorrichtung kann die Blutpumpe 34 auch bei und nach Schließen der Klemme 40 für die Erkennung einer Nadeldiskonnektion weiter fördern und es wird der Behandlungsverlauf zumindest auf der arteriellen Seite durch die Messung nicht beeinträchtigt.

Die Überwachung des extrakorporalen Blutkreislaufs zur Erkennung einer venösen Nadeldiskonnektion kann bei Blutbehandlungsvorrichtungen, die im Ein-Nadel-Betrieb arbeiten, und ebenso auch bei Blutbehandlungsvorrichtungen erfolgen, die im Zwei-Nadel-Betrieb mit einer arteriellen und einer venösen Kanüle zur Herstellung des Patientenzugangs arbeiten.

Mit der Erfindung wird somit eine Vorrichtung zur Erkennung einer Nadeldiskonnektion am Gefäßzugang, z.B. dem venösen Gefäßzugang, geschaffen. Diese Vorrichtung kann beispielsweise bei einer Vorrichtung zur extrakorporalen Blutbehandlung, insbesondere einer chronischen Blutreinigungstherapie wie Hämodialyse, Hämofiltration und Hämodiafiltration eingesetzt werden. Damit kann die Überwachung des Gefäßzugangs während der extrakorporalen Blutbehandlung erfolgen, und sowohl eine Nadeldiskonnektion als auch eine Nadeldislozierung am venösen Gefäßzugang sicher erkannt werden. Auch ein Blutleck, z.B. im venösen Zweig, in Flussrichtung hinter der Klemme 40, des extrakorporalen Kreislaufs ist zuverlässig detektierbar.

Durch die Schlauchabsperrung 40 ist die Rückführung des gereinigten, aus dem Dialysator 1 austretenden Bluts zum Patienten absperrbar oder zumindest drosselbar.

In Blutflussrichtung gesehen kann bei einem Ausführungsbeispiel die Schlauchabsperrung 40 das vorletzte Bauteil bilden und der Drucksensor 41 das letzte Bauteil der Vorrichtung zur extrakorporalen Blutbehandlung darstellen. Durch die Schlauchabsperrung 40 ist es möglich, auch bei laufender Blutpumpe 34 den Fluss vom Drucksensor 41 bis zur Rückführung in den Patienten durch Betätigung der Schlauchabsperrung 40 zu stoppen oder zumindest zu drosseln.

Der Drucksensor 41 muss nicht zwingend das letzte Bauteil vor der Rückführung des Blutes zum Patienten sein. Vorzugsweise sind aber zwischen dem Drucksensor 41 und der Eintrittsstelle des gereinigten Bluts in den Patientenkreislauf keine weiteren Bauteile mehr vorhanden, die den zeitlichen Verlauf des durch den Drucksensor 41 gemessenen Drucks während der Messung bei geschlossener Schlauchabsperrung 40, z. B. Schlauchabsperrklemme, in merklicher oder nicht vorhersehbarer Weise deutlich beeinflussen. Bei einem, mehreren oder allen Ausführungsbeispielen befindet sich die Schlauchabsperrung 40 in Blutflussrichtung gesehen vor dem Drucksensor 41. Bei einem, mehreren oder allen Ausführungsbeispielen befinden sich, gesehen in Blutflussrichtung, ausgehend von dem Dialysator 1 entlang der Blutabführleitung 31, zunächst alle anderen Bauteile oder Komponenten, insbesondere solche, die den Druck beeinflussen können, wie z.B. Blasenfänger, Drucksensoren, Messeinrichtungen usw., wobei die Schlauchabsperrung 40 als vorletztes dieser Bauteile positioniert ist und der Drucksensor 41 bei diesen oder anderen Ausführungsbeispielen als letztes Bauteil des Ausführungsbeispiels der erfindungsgemäßen Vorrichtung angeordnet ist, wonach in Blutflussrichtung gesehen nur noch die in den Patienten führende Nadel folgt, deren ordnungsgemäße Positionierung und Fluidverbindung detektiert werden soll (Diskonnektion).

Die Schlauchabsperrung 40 und der Drucksensor 41 sind mit der Steuereinrichtung 50 verbunden, die der Schlauchabsperrung 40 Befehle zur Sperrung und Freigabe der Fluidströmung zuführt und den durch den Drucksensor 41 gemessenen Druck nach der Absperrung misst und hieraus erkennt, ob eine ordnungsgemäße Verbindung zwischen einem Dialysesystem, insbesondere einer Nadel des Dialysesystems, und dem Patienten-Blutkreislauf vorliegt oder nicht.

Ein weiteres Ausführungsbeispiel der Erfindung stellt eine Vorrichtung zur extrakorporalen Blutbehandlung bereit, die einen Dialysator 1, eine Schlauchabsperrung 40, einen Drucksensor 41, eine Datenverarbeitungseinheit, eine Speichereinheit, die ggf. Bestandteil der Datenverarbeitungseinheit sein kann, und eine Ausgabeeinheit aufweist. Der Dialysator 1 kann hierbei durch eine semipermeable Membran in eine erste und eine zweite Kammer 29, 30 unterteilt sein, wobei die erste Kammer 29 in einem Dialysierflüssigkeitsweg angeordnet ist und die zweite Kammer 30 mittels einer Blutzuführleitung 32 und einer Blutabführleitung 31 des Dialysators 1 mit dem Blutkreislauf eines Patienten verbindbar ist. Weiterhin kann ggf. ein Blasenfänger 35, 38 vorgesehen sein. Die Schlauchabsperrung 40 und der Drucksensor 41 sind vorzugsweise in der Blutabführleitung 31 des Dialysators 1 nach dem, d.h. stromab des, Blasenfängers 38 angeordnet.

Das in Fig. 1 gezeigte Ausführungsbeispiel stellt einen möglichen Aufbau einer erfindungsgemäßen Vorrichtung zur extrakorporalen Blutbehandlung und/oder zur Erkennung einer Nadeldiskonnektion dar. Bei einem anderen Ausführungsbeispiel kann beispielsweise auch auf den Drucksensor 37 verzichtet werden, da dessen Funktion durch den bei diesem Ausführungsbeispiel vorgesehenen und gemäß der Darstellung in Fig. 1 angeordneten Drucksensor 41 übernommen werden kann.

Anhand der durch den Drucksensor 41 erfassten Messdaten wird bei dem Ausführungsbeispiel gemäß Fig. 1 eine regelmäßige Prüfung auf venöse Nadeldiskonnektion durchgeführt. Die regelmäßige Prüfung kann als Einzelprüfung oder Mehrfachprüfung durchgeführt werden. Eine Mehrfachprüfung besteht aus mindestens zwei Einzelprüfungen. Ebenso können dreifache, vierfache bis hin zu n-fachen Prüfungen während einer Dialyse durchgeführt werden, wobei n eine natürliche Zahl größer oder gleich 5 ist.

Die Prüfung auf eine venöse Nadeldiskonnektion erfolgt, indem die Schlauchabsperrung 40 geschlossen wird, sodass in die Blutabführleitung 31, über die das Blut wieder in den Patienten zurückgeführt wird, kein Blut mehr nachfließen kann. Als Schlauchabsperrung 40 können beliebige Vorrichtungen dienen, welche den Fluss durch die als Schlauch ausgebildete Blutabführleitung 31 unterbinden, wie z.B. Schlauchabsperrklemmen oder Ventile.

Bei abgeklemmtem Schlauch 31 erfolgt dann am Drucksensor 41 eine Druckmessung kontinuierlich oder in, vorzugsweise regelmäßigen, zeitlichen Intervallen so lange, bis anhand der gemessenen Druckwerte festgestellt werden kann, ob diese gegen Atmosphärendruck tendieren.

Atmosphärendruck bedeutet hier, dass der Druck, gegen den der Druckverlauf nach Schließen der Schlauchabsperrung tendiert, nicht mehr den Shunt- oder Gefäßinnendruck beinhaltet oder wiedergibt, sondern das System über die die Blutabführleitung 31 mit dem Patienten verbindende Nadel gegen Atmosphäre geöffnet ist. In diesem Fall ist der Druck nicht mehr durch den Gegendruck im Patientenzugang bedingt. Es tritt ein Flüssigkeitsverlust über die Nadel auf, bis der Druck innerhalb des Schlauchsystems dem Umgebungsdruck der Nadel entspricht, der entweder der Atmosphärendruck oder der Druck im venösen Patientenzugang, jeweils unter Berücksichtigung des hydrostatischen Drucks durch einen möglichen Höhenunterschied zwischen dem Flüssigkeitspegel im Luftblasenfänger 38 und dem venösen Zugang ist.

Das Erreichen der unterschiedlichen Druckniveaus, das abhängig ist von dem am Auslass der zum Patientenzugang führenden Nadel herrschenden Druck (Atmosphärendruck oder Patienteninnendruck), spiegelt sich auch in der Geschwindigkeit wider, mit der die Druckänderung nach Stoppen des Flusses durch Sperren der Schlauchabsperrung 40 in der Messvorrichtung vonstatten geht. Hierbei kann die Dauer des Abfalls des Drucks z. B. auf 36,8 % des bei laufendem Blutfluss, d.h. geöffneter Schlauchabsperrung 40, vorhandenen Werts (1/e des Werts) als Maß verwendet werden.

Zusätzlich oder alternativ kann optional überprüft werden, ob nach Schließen der Schlauchabsperrung 40 ein periodisches Signal nachgewiesen werden kann, welches dem Drucksignal überlagert ist und durch den pulsierenden Blutdruck im Patientenzugang erzeugt wird. Kann ein solches pulsierendes periodisches Signal nicht mehr nachgewiesen werden, ist dies ebenfalls ein Indiz für eine venöse Nadeldiskonnektion, da keine Verbindung zum Patientenkreislaufsystem mehr vorliegt. Wird eine Nadeldiskonnektion erkannt, wird vorzugsweise ein Warnsignal ausgegeben. Bei einem oder mehreren Ausführungsbeispielen bleibt die Schlauchabsperrung 40 in einem solchen Fall geschlossen, sodass ein unerwünschtes Abströmen des Bluts in die gegenüber der Atmosphäre freiliegende oder in das Patientengewebe unerwünscht führende Nadel verhindert wird. Während der Prüfung auf eine venöse Nadeldiskonnektion bleibt die Schlauchabsperrung 40 geschlossen.

Wenn bei der Überprüfung auf Nadeldiskonnektion erkannt wird, dass keine Nadeldiskonnektion vorliegt, d.h. die Dialysemaschine korrekt an den Blutkreislauf des Patienten angeschlossen ist, wird die Schlauchabsperrung 40 wieder geöffnet und die Dialyse fortgeführt, bis nach einem vorgegebenen Zeitintervall eine erneute Prüfung auf eine venöse Nadeldiskonnektion durchgeführt wird. Die Zeitabstände zwischen den Prüfungen auf venöse Nadeldiskonnektion können automatisch ermittelt werden oder einstellbar sein, z.B. für zu unkontrollierten Bewegungen neigenden Risikopatienten. Bei Ausführungsbeispielen der erfindungsgemäßen Vorrichtung liegt der Zeitabstand zwischen den Prüfungen im Bereich von 2 Sekunden bis 5 Minuten, oder im Bereich von 10 Sekunden bis 4 Minuten, oder im Bereich von 20 Sekunden bis 3 Minuten, oder im Bereich von 25 Sekunden bis 120 Sekunden, oder auch im Bereich zwischen 30 und 60 Sekunden, sodass ein oder zwei Mal je Minute eine Überprüfung durchgeführt wird.

Eine automatische Ermittlung der Zeitabstände zwischen den Prüfungen kann bei einem oder mehreren Ausführungsbeispielen abhängig vom Patientengewicht und eingestelltem Blutfluss erfolgen. Es können ca. 13 % des Gesamtblutvolumens des Patienten entzogen werden, bevor ein Effekt auf den arteriellen Druck des Patienten oder auf dessen Herzminutenvolumen (cardiac output) auftritt. So kann beispielsweise bei einem Ausführungsbeispiel bei Kenntnis des Blutvolumens des Patienten von beispielsweise 6 Litern und einer Blutflussrate von 300 ml/min die Abtastfrequenz auf 0,0083 Hz, also auf eine Messung alle 2 Minuten, bestimmt werden. Kritisch wird der Blutverlust, wenn 35 bis 45 % des Blutvolumens entfernt werden. Hieraus lässt sich ein absoluter Minimalwert für die Abtastfrequenz bestimmen. Weitere abhängige Parameter können Herz- und/oder Atemfrequenz, Begleiterkrankungen wie z.B. Diabetes mellitus und/oder koronare Herzerkrankungen sein. Eine einstellbare Einzelprüfung kann beispielsweise nach einem ungewöhnlichen, unspezifischen Druckabfall am Drucksensor 37 manuell durchgeführt oder automatisch ausgelöst werden. Wird beispielsweise am Drucksensor 37 ein Druckabfall von mehr als einem Referenzwert von beispielsweise 10 % bis 30 % oder mehr als des durchschnittlichen, durch den Drucksensor 37 erfassten Drucks ermittelt, wird die Einzelprüfung ausgelöst.

Bei Ausführungsbeispielen der erfindungsgemäßen Vorrichtung kann als weiteres Auslösekriterium (Trigger) für den Start einer neuen oder zusätzlichen Messung die Erkennung eines starken Abfalls im venösen Druck zwischen den Messintervallen während einer laufenden Behandlung verwendet werden. Dies bedeutet, dass bei einer kontinuierlichen Druckmessung am Drucksensor 41 bei geöffneter Schlauchabsperrung 40 dann automatisch eine neue Messung gestartet wird, wenn bei dieser kontinuierlichen Druckmessung am Drucksensor 41 größere Druckschwankungen oder ein Druckabfall von beispielsweise mehr als 20 % des bislang vorhandenen durchschnittlichen Drucks detektiert werden. Der Start einer neuen Messung bedeutet auch hier, dass die Schlauchabsperrung 40 geschlossen wird und am Drucksensor 31 gemessen wird, ob der Druck gegen Atmosphärendruck tendiert.

Im Folgenden werden die Arbeitsweise des gezeigten und beschriebenen Ausführungsbeispiels sowie eine Ausführungsform der erfindungsgemäßen Vorrichtung zur Überprüfung und/oder Erkennung des Vorliegens einer venösen Nadeldiskonnektion beschrieben. Mit "venöser Nadeldiskonnektion" ist auch hier eine Verlagerung einschließlich eines Herausrutschens der von der Blutabführleitung 31 in den Patienten führenden Nadel bzw. eine unbeabsichtigte Auftrennung oder Öffnung der Verbindung der Blutabführleitung 31 mit dem Patientenzugang, beispielsweise in Form eines Bajonettverschlusses, Drehverschlusses oder Steckverschlusses, zu verstehen. Für die Durchführung der Überprüfung auf venöse Nadeldiskonnektion wird zunächst die Schlauchabsperrung 40, beispielsweise in Form einer Schlauchabsperrklemme, geschlossen. Alternativ oder zusätzlich kann die Blutpumpe 34 angehalten werden. Der an dem Drucksensor 41 nach Schließen der Schlauchabsperrung 40 auftretende Druckabfall wird mit einer ausreichenden Auflösungs- und/oder Abtastrate aufgenommen, wobei die hierbei ermittelten Messwerte des Druckabfalls bzw. des jeweils gemessenen Drucks in der Auswerte- und Speichereinheit 50 abgelegt werden. Die Speichereinheit kann als interner Speicher, beispielsweise als Arbeitsspeicher, oder auch als externer Speicher ausgeführt sein. Bei einem oder mehreren Ausführungsbeispielen liegt die hier auch als Abtastrate bezeichnete Auflösungsrate im Bereich von 1 bis 2000 Messungen pro Sekunde oder im Bereich von 2 bis 1000 Messungen pro Sekunde, oder im Bereich von 3 bis 500 Messungen pro Sekunde, oder im Bereich von 4 bis 200 Messungen pro Sekunde, oder im Bereich von 5 bis 100 Messungen pro Sekunde, oder im Bereich von 6 bis 50 Messungen pro Sekunde. Hierdurch wird gewährleistet, dass der Druckverlauf mit einer für die Analyse ausreichenden Qualität und Genauigkeit abgebildet wird.

Fig. 2 zeigt ein Beispiel für den Druckverlauf am Drucksensor 41 vor und nach einer Nadeldiskonnektion. Der Druck am Drucksensor 41, d.h. der von diesem gemessene Druck, stellt sich in Höhe des Gefäßdrucks am Patientenzugang, d.h. beispielsweise im Shunt, ein. Ein Druck von 25 mmHg entspricht hier dem durchschnittlichen Shuntinnendruck eines AV-Shunts (arteriovenöser Shunt), der aus autologem Material besteht. Der durchschnittliche Shuntinnendruck aller Dialysepatienten mit nativem AV-Shunt und AV-Shunts mit PTFE-Prothese liegt bei ca. 35 mmHg. Ein Wert von ca. 40 mmHg entspricht dem Mittel von 80 % aller Dialysepatienten mit nativem AV-Shunt. Unter 70 mmHg liegen im Mittel die Shuntinnendrücke von 80 % aller Dialysepatienten mit PTFE-Prothese.

Im Folgenden wird exemplarisch ein Druck von 30 mmHg angenommen. Dies liegt somit in der Größenordnung der typischen Druckpulse, die durch die Blutpumpe 34 bei laufender Therapie erzeugt werden.

Bei dem Ausführungsbeispiel gemäß Fig. 3 erfolgt die Erfassung einer eventuellen venösen Nadeldiskonnektion bei gestopptem Blutfluss. Dies kann durch das Anhalten einer im arteriellen Teil des extrakorporalen Kreislaufs angeordneten Blutpumpe 34, oder auch durch das Abklemmen des Schlauchsystems bei laufender Blutpumpe 34 mittels der Schlauchklemme 40 erfolgen. Bei einem weiteren Ausführungsbeispiel können sowohl die Blutpumpe 34 als auch die Schlauchklemme 40 gemeinsam angesteuert werden, sodass sowohl die aktive Fluidförderung durch die Blutpumpe 34 als auch die Weiterleitung des Bluts durch die Schlauchklemme 40 hindurch gleichzeitig, oder auch zeitversetzt, beendet werden. Bei einem gestoppten Blutfluss läuft der Druck im gesamten extrakorporalen Blutschlauchsystem gegen den Shunt- oder Gefäßinnendruck des Patienten. Fig. 3 zeigt einen typischen Kurvenverlauf für den am Drucksensor 41 auftretenden und durch diesen gemessenen Druck ohne venöse Nadeldiskonnektion, wobei die Messung zu einem Zeitpunkt $t_1$ begonnen und zu einem Zeitpunkt $t_2$ wieder beendet wird.

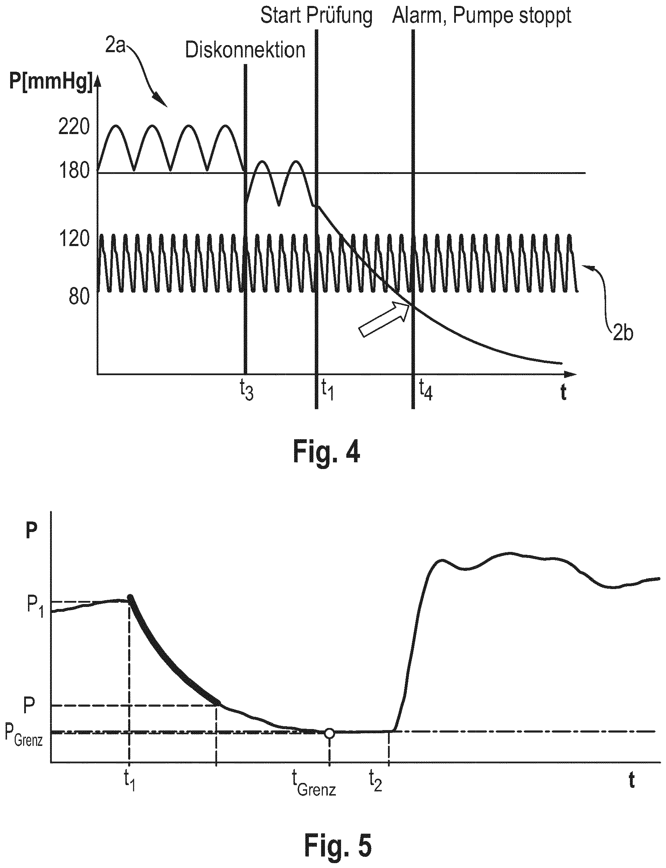

Beim Ausführungsbeispiel gemäß Fig. 4 tritt eine unerwünschte Nadeldiskonnektion bzw. Auftrennung der Verbindung zwischen dem extrakorporalen Kreislauf und dem Patientenzugang zu einem Zeitpunkt $t_3$ auf. Diese Nadeldiskonnektion bzw. Auftrennung der Verbindung zum Patientenzugang wird bei einer regelmäßigen, zum Zeitpunkt $t_1$ gestarteten Prüfung detektiert und führt nach Absinken des gemessenen Blutdrucks unter einen Schwellwert $p_{Grenz}$ zu dem Zeitpunkt $t_4$ zur Auslösung eines Alarms und ggf. zusätzlich zum Anhalten der Blutpumpe 34, falls diese noch laufen sollte. Der Schwellenwert $p_{Grenz}$ kann beispielsweise dadurch ermittelt werden, dass bei sicher angelegtem venösen Zugang eine Prüfung durchgeführt wird. Bei Unterschreiten des Schwellenwerts $p_{Grenz}$ wird zusätzlich oder alternativ zur Auslösung eines Warnsignals der Dialysevorgang unterbrochen und die Vorrichtung zur extrakorporalen Fluidbehandlung geht in einen sicheren Zustand ohne weitere Fluidentnahme über, um dem Patienten keinen Schaden zuzufügen.

Eine venöse Nadeldiskonnektion wird dadurch detektiert, dass ab Beginn der Prüfung zum Zeitpunkt $t_1$ zeitversetzt, d.h. in zeitlichem Abstand, Druckmesswerte am Drucksensor 41 gemessen werden und überprüft wird, ob diese Druckmesswerte in ihrem Abfall gegen Atmosphärendruck tendieren und beispielsweise den Schwellenwert $p_{Grenz}$ erreichen und unterschreiten. Zeigen die am Drucksensor 41 gemessenen Druckmesswerte eine Tendenz gegen Atmosphärendruck, d.h. eine relativ rasche Abnahme oder ein Erreichen und Unterschreiten des Schwellenwerts $p_{Grenz}$, wird auf das Vorliegen einer venösen Nadeldiskonnektion bzw. einer unerwünscht fehlerhaften Verbindung zwischen dem Blutabführschlauch 31 und dem Patientenzugang, beispielsweise dem Shunt, geschlossen. Tendieren hingegen die am Drucksensor 41 gemessenen Druckmesswerte zu einem typischen venösen Druckniveau und zeigen demgemäß nur eine geringe Abnahmerate bzw. erreichen oder unterschreiten den Schwellenwert $p_{Grenz}$ nicht, so liegt keine venöse Nadeldiskonnektion vor, und es wird die Prüfung zu dem Zeitpunkt $t_2$ (Fig. 3) beendet und die Behandlung, d.h. die Dialyse, fortgesetzt. Dieser Fall ist in Fig. 3 dargestellt.

Somit kann beispielsweise bei laufender Blutpumpe 34 und geöffneter Schlauchabsperrung 40 am Drucksensor 41 ein Druck von 160 mmHg gemessen werden. Zeigen die nachfolgenden Druckmessungen bei geschlossener Schlauchabsperrung 40 am Drucksensor 41 einen Druckabfall, der gegen Atmosphärendruck tendiert, d.h. auf einen Wert von 0 mmHg relativ zum Atmosphärendruck, liegt eine venöse Nadeldiskonnektion vor und es wird eine Warnhinweis ausgegeben, beispielsweise in optischer Form als visuelle Anzeige oder in akustischer Form durch Generierung eines akustischen Alarms, und es wird ggf., sofern noch nicht erfolgt, die Blutpumpe 34 gestoppt. Tendieren hingegen die am Drucksensor 41 nach dem Beginn der Messung ermittelten Werte des Drucksensors beispielsweise gegen 30 mmHg, so liegt dieser Tendenzwert im Bereich eines typischen venösen Druckniveaus, sodass entschieden wird, dass keine venöse Nadeldiskonnektion vorliegt.

Bei einem oder mehreren Ausführungsbeispielen der erfindungsgemäßen Vorrichtung ist eine Vorhersage des Kurvenverlaufs nach dem Schließen der Schlauchabsperrung 40, z.B. der Schlauchabsperrklemme, vorgesehen, um die Dauer der Prüfung zu verringern. Dies kann bei dem oder den Ausführungsbeispielen dadurch erfolgen, dass anhand der ersten Messwerte nach Start der Prüfung zum Zeitpunkt $t_1$ ein Wert extrapoliert wird, gegen den der Druckverlauf tendiert. Anhand der Größe des Druckabfalls nach Schließen der Schlauchabsperrung 40 lässt sich eine relativ zuverlässige Vorhersage treffen, ob der Druckabfallgradient gegen den venösen Blutdruck des Patienten oder aber gegen Atmosphärendruck tendiert.

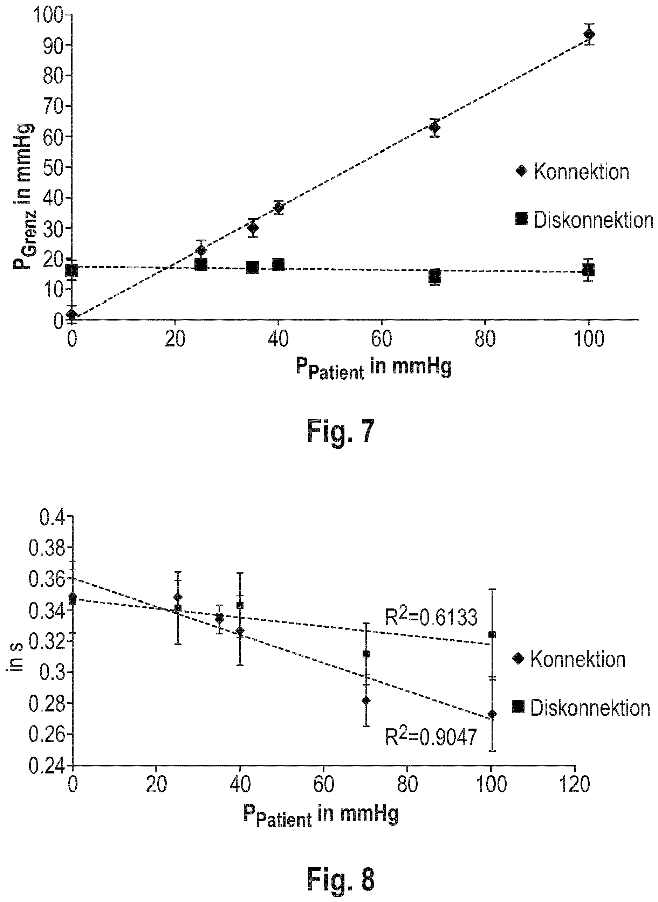

Anstelle oder zusätzlich zu der Analyse des Drucks am Drucksensor 41 bei geschlossener Schlauchabsperrung 40 kann auch der Verlauf des Druckabfalls zur Erkennung einer venösen Nadeldiskonnektion herangezogen werden. Bei einem Ausführungsbeispiel wird z.B. die Dauer τ des Abklingens des Drucks auf nur noch 36,8 %, d.h. auf 1/e, als Kenngröße herangezogen. Diese Kenngröße unterscheidet sich für die Fälle der Konnektion bzw. der Diskonnektion signifikant (Fig. 8), sodass auch hieraus zuverlässig eine eventuelle Nadeldiskonnektion erkannt werden kann. Die Steuereinrichtung 50 überprüft in diesem Fall die Zeitdauer τ, die zum Erreichen eines Druckabfalls auf 1/e oder einen anderen Vorgabewert erforderlich ist, und vergleicht diese Zeitdauer τ ggf. mit einem zeitlichen Schwellenwert oder Referenzwert. Liegt die gemessene Dauer τ unterhalb des zeitlichen Schwellenwerts, wird auf Diskonnektion geschlossen. Andernfalls wird auf den Fall ordnungsgemäßer Konnektion geschlossen und die Behandlung fortgesetzt. Auch dieser Ansatz stellt ein geeignetes Mittel dar, um eine venöse Nadeldiskonnektion zu erkennen.

Bei den beschriebenen oder anderen Ausführungsbeispielen kann alternativ oder zusätzlich überprüft werden, ob nach Schließen der Schlauchabsperrung 40 weiterhin ein periodisches Signal am Drucksensor 41 gemessen wird. Ist dies der Fall, liegt keine Diskonnektion vor, da das periodische Signal durch den pulsatilen Blutdruck erzeugt wird. Die Messung kann in diesem Fall direkt abgebrochen und die Dialyse fortgesetzt werden. Diese Vorgehensweise zeichnet sich dadurch aus, dass die Dialyseunterbrechung äußerst kurzfristig ist. Erhält man demgegenüber einen Abfall der Druckkurve ohne ein aufgeprägtes periodisches Signal, ist eine Diskonnektion möglich oder wahrscheinlich. In diesem Fall wird die Messung bei dem beschriebenen Ausführungsbeispiel fortgesetzt, bis ein eindeutiges Ergebnis erzielt ist. In manchen Fällen bzw. bei einigen Ausführungsbeispielen kann auch vorgesehen sein, die Blutpumpe 34 komplett anzuhalten, beispielsweise wenn das extrakorporale Speichervolumen, das durch den oder die Blasenfänger 35, 38 oder andere Komponenten bereitgestellt wird, ausgeschöpft ist. In allen diesen Fällen ist die Patientensicherheit gewährleistet.

Alternativ oder zusätzlich kann während der Prüfung auf venöse Nadeldiskonnektion die Blutpumpe 34 weiter fördern. Dies führt zu einer Schwankung des Füllstandspegels im venösen Blasenfänger 38 und/oder im arteriellen Blasenfänger 35, in dem die Menge des in dieser Zeit geförderten Blutes zwischengespeichert wird. Fig. 3 zeigt, dass der Druck in dem venösen, das Patientenblut wieder zuführenden Schlauch (Leitung) 31 nach dem Öffnen der Schlauchabsperrung 40 gemessen wird, was zu einem Druckanstieg am Drucksensor 41 führt. Im Anschluss hieran fällt der Druck wieder auf das Grundniveau, nicht aber tiefer ab. Diese Vorgehensweise bringt den Vorteil mit sich, dass keine Dialysezeit für die Prüfung auf venöse Nadeldiskonnektion vergeudet wird, da die Dialyse während dieses Messzeitraums weitergeführt werden kann.

Zur Bestimmung des Endpunkts der Druckmessung am Drucksensor 41 kann ein Fit, bzw. ein Schwellenwert oder Sollkurvenverlauf verwendet werden. Als Verlauf wird vorzugsweise ein exponentieller Abfall des Drucks gemäß der folgenden Formel angenommen:

$$F(t) = e^{(-at)} + A$$

Hierbei bezeichnet A einen Offsetwert, also denjenigen Wert, gegen den die Funktion konvergiert, wohingegen mit a die Abklingkonstante bezeichnet ist. Dies stellt nur eines von mehreren möglichen Ausführungsbeispielen der Umsetzung dar. Das Verhalten bzw. die für den Vergleich angesetzten Größen können auch durch Bi-Exponentialfunktionen und Polynome beschrieben werden.

Der Fit bzw. der Schwellenwert bzw. die Referenzkurve kann im Verlauf der Messung bzw. während der Messzeit dynamisch angepasst werden. Es werden hierbei bei diesem Ausführungsbeispiel neue Datenpunkte, d.h. neue aktuell ermittelte Messwerte, sofort mit in den Fit integriert, d.h. die Sollkurve oder der Schwellenwert wird angepasst, um die Vorhersage des zu erwartenden Endwertes so präzise wie möglich zu machen. Das Einbeziehen zusätzlicher Informationen in Form neuer Werte kann auch den berechneten Endwert der Fitfunktion bzw. Referenzfunktion beeinflussen. Dieser ist bei dieser Vorgehensweise also einer gewissen Schwankung ausgesetzt. Diese Schwankung kann als zusätzlicher Kontrollparameter für die Verlässlichkeit der Endwertvorhersage verwendet werden. Je kleiner die Schwankung ist, desto verlässlicher ist die Aussage, desto genauer ist sie also. Liegt die ermittelte Schwankung innerhalb eines Intervalls von weniger als 20 mmHg, vorzugsweise weniger als 10 mmHg, oder von weniger als 5 mmHg, wird die Messung als abgeschlossen bewertet.

Unter dem Endpunkt der Druckmessung ist hier derjenige Druckwert zu verstehen, den der vom Drucksensor 41 gemessene Druck laut Vorhersage des Fits nach dem Schließen der Schlauchabsperrung 40 erreichen würde.

Die beschriebenen Ausführungsbeispiele der erfindungsgemäßen Vorrichtung zur Prüfung auf venöse Nadeldiskonnektion zeichnen sich dadurch aus, dass der venöse Druck vom arteriellen Druck im extrakorporalen Kreislauf entkoppelt wird und nicht einfach die Druckdifferenz zwischen venösem und arteriellem Druck bestimmt wird. Der arterielle Druckverlauf stellt daher keine störende Größe dar, und es führen Bewegungen des Patienten oder eine Verstellung der Liegehöhe oder Liegeposition des Patienten nicht zu einem Fehlalarm. Dies liegt daran, dass bei Ausführungsbeispielen der erfindungsgemäßen Vorrichtung ausschließlich ein Druckabfall im venösen Druck hinter einer, bzw. stromab einer, Schlauchabsperrung gemessen und beobachtet wird, ob der Druckabfall gegen Atmosphärendruck oder ein im venösen Bereich übliches Druckniveau tendiert. Damit kann eine fehlerfreie und präzise Diagnose getroffen werden.

Im Folgenden wird ein konkretes Ausführungsbeispiel beschrieben. Zu Beginn einer Dialyse wird zunächst ein Druckwert durch den Drucksensor 41 bei unterbrochenem Fluss (Schließung der Schlauchabsperrung 40) und unter Gewährleistung eines ordnungsgemäß angelegten venösen Patientenzugangs als Referenzwert aufgenommen. Dieser beträgt hier beispielhaft 35 mmHg. Während der ersten Behandlungsphase von beispielsweise den ersten beiden Behandlungsstunden wird nun in Intervallen von beispielsweise 2 Minuten die Schlauchabsperrung 40 geschlossen und der Druckverlauf am Drucksensor 41 aufgezeichnet. Eine beim Ausführungsbeispiel durchgeführte Extrapolation des über einen bestimmten Zeitraum von beispielsweise 1,5 s aufgenommenen Druckverlaufs ergibt, dass der Gegendruck an der venösen Nadel je nach Messung zwischen beispielsweise 30 mmHg und 40 mmHg liegt. Damit kann die Schlauchabsperrung 40 umgehend wieder geöffnet werden, um die Behandlung des Patienten regulär fortzusetzen. Nach jeder Messung wird das zwischengespeicherte Blutvolumen, das durch die laufende Blutpumpe 34 bei geschlossener Schlauchabsperrung 40 im Luftblasenfänger 38 gesammelt wurde, allmählich wieder in den Patienten zurückgegeben. Um Druckspitzen durch das Öffnen der Schlauchabsperrung 40 zu vermeiden, kann ein Druckausgleich in den Luftblasenfängern 38 und 35 vorgenommen werden. Hierfür ist beispielsweise eine Pumpe geeignet, die zum Einstellen der Flüssigkeitspegel in den Luftblasenfängern 35, 38 verwendet wird.

Auch während dieses vorstehend geschilderten Vorgangs läuft die Blutpumpe 34 mit der vom Anwender gewählten Förderrate von beispielsweise 300 ml/min weiter, sodass der mittlere Blutfluss, über die komplette Behandlung hinweg betrachtet, dem durch den Anwender gewählten Blutdruck entspricht.

Bei der Fortsetzung der Behandlung nach der Messung, also bei dem beschriebenen Beispiel in der dritten Behandlungsstunde, tritt nun bei diesem Ausführungsbeispiel eine venöse Nadeldiskonnektion auf. Bei der nachfolgenden Messung bei geschlossener Schlauchabsperrung 40 wird ein Druckverlauf am Drucksensor 41 detektiert, der gegen den Atmosphärendruck läuft (0 mmHg Differenzdruck). Die venöse Nadel ist beispielsweise auf dem Patientenbett liegen geblieben und befindet sich in Höhe des Druckaufnehmers 41. Sobald dies erkannt wird, wird sofort ein Alarm ausgelöst und ggf. wird die Blutbehandlung unterbrochen, indem die Blutpumpe 34 angehalten wird. Das Behandlungspersonal kann sich nun zu dem Patienten begeben und die Situation beheben. Anschließend kann die Behandlung fortgesetzt werden.

Einen gewissen Zeitraum von beispielsweise 40 Minuten später wird ein Druck von beispielsweise -70 mmHg gemessen, woraufhin die Blutpumpe 34 gestoppt und ein Alarm abgegeben wird. Bei diesem Beispiel ist die Nadel nicht auf dem Bett liegen geblieben, sondern auf den Boden gefallen. Damit befindet sich die Nadel bei diesem Beispiel nun unterhalb des Druckaufnehmers 41. Die hydrostatische Säule zwischen Druckaufnehmer 41 und Öffnung gegen Atmosphäre (venöse Nadel am Boden) erzeugt nun also einen negativen Druck im Drucksensor 41.

Nach einem weiteren Zeitraum von beispielsweise 20 Minuten wird bei einer nachfolgenden Messung bei der Extrapolation des Druckverlaufs aus der z.B. 1,5 Sekunden lang dauernden Messung kein eindeutiges Ergebnis ermittelt. Zeitgleich mit der Messung hat beispielsweise der Patient seine Armposition geändert und somit eine starke Schwankung in der Extrapolation des Druckverlaufs hervorgerufen. Zunächst wird nun die Messung nach einem bestimmten Zeitraum von beispielsweise 15 Sekunden später erneut wiederholt, um das Ergebnis der vorherigen Messung zu überprüfen. Da bei diesem Beispiel erneut keine eindeutige Aussage aus den Messergebnissen nach beispielsweise 1,5 Sekunden getroffen werden kann, bleibt die Schlauchabsperrung 40 geschlossen, bis ein eindeutiges Ergebnis vorliegt. In einem solchen Fall kann zusätzlich zu dem zunächst für die Zwischenspeicherung des gereinigten Bluts eingesetzten Blasenfänger 38 nun auch der Blasenfänger 35 als Blutreservoir für noch ungereinigtes Blut hinzugezogen werden, wobei der Dialysator 1 in diesem Fall in seinem Fördervolumen gedrosselt oder ganz auf Null heruntergefahren werden kann. Das bei weiterlaufender Blutpumpe 34 geförderte Blutvolumen wird also nun im Blasenfänger 35 zwischengespeichert. Ist das Ergebnis immer noch nicht mit ausreichender Genauigkeit behaftet, wird die Blutpumpe 34 in diesem Fall ebenfalls angehalten, bis ein eindeutiges Ergebnis erzielt ist. Bei dem beschriebenen Beispiel ist allerdings nach einer Messzeit von beispielsweise 6 Sekunden eindeutig, dass keine venöse Nadeldiskonnektion vorliegt, da der extrapolierte Wert nun bei 35 mmHg liegt, also bei demjenigen Wert, der auch zu Beginn der Behandlung als Referenz für einen ordnungsgemäßen Zugang aufgenommen und gespeichert wurde. Die Behandlung wird damit fortgesetzt.

Wie vorstehend beschrieben, zeigt Fig. 3 einen schematischen Druckverlauf am Drucksensor 41 bei korrekt positionierter venöser Nadel 31a. Die obere Kurve 2a symbolisiert auch hier den venösen Druckverlauf, während die untere, gleichförmige Kurve 2b den venösen Blutdruck im Körper des der Blutbehandlung zu unterziehenden Patienten angibt. Bei einem Zeitpunkt $t_1$, "Start Prüfung", wird die Schlauchabsperrung 40 geschlossen und der Drucksensor 41 misst, ob weiterhin ein durch den pulsierenden Blutdruck des Patienten erzeugtes periodisches Signal vorliegt und/oder ob die durch den Druckmesser 41 gemessenen Druckmesswerte gegen Null tendieren.

Aus dem in Fig. 3 gezeigten Verlauf ist erkennbar, dass der Druckabfall nicht gegen Null, sondern gegen ca. 80 mmHg tendiert und ferner weiterhin ein periodisches Signal durch den pulsatilen Blutdruck vorliegt. Hieraus wird erkannt, dass keine Nadeldiskonnektion vorliegt, und es wird die Blutbehandlung nach dem Ende der Prüfung zum Zeitpunkt $t_2$ fortgesetzt. Hierbei wird die Schlauchabsperrung 40 wieder geöffnet, wobei es durch den vor (stromauf) der Schlauchabsperrung 40 aufgebauten Druck zu einem Druckmaximum (Überschwinger) kommen kann, wie es im Druckkurvenverlauf 2a unmittelbar nach dem Zeitpunkt $t_2$ erkennbar ist.

Fig. 4 zeigt ein Beispiel eines Druckverlaufs am Drucksensor 41 der erfindungsgemäßen Vorrichtung bei einer venösen Nadeldiskonnektion. Die obere Kurve 2a symbolisiert auch hier den venösen Druckverlauf, während die untere Kurve 2b den venösen Blutdruck im Körper des zu dialysierenden Patienten anzeigt. Bei dem Beispiel gemäß Fig. 4 ist zum Zeitpunkt $t_3$ eine Nadeldiskonnektion, d.h. eine Auftrennung der Verbindung zwischen der Blutbehandlungsvorrichtung und dem Patientenblutkreislauf, beispielsweise durch Herausrutschen der venösen Nadel oder durch Auftrennung der in sonstiger Weise hergestellten Fluidverbindung zwischen der Dialysevorrichtung und dem Patientenblutkreislauf, aufgetreten. Die Schlauchabsperrung 40 wird zum Zeitpunkt $t_1$, "Start Prüfung", geschlossen. Zwei Pumpenschläge vor dem Zeitpunkt $t_1$, d.h. dem Start der Prüfung, war zum Zeitpunkt $t_3$ tatsächlich eine Nadeldiskonnektion aufgetreten. Nun tendiert der Druck in der Blutabführleitung 31 nach dem Schließen der Schlauchabsperrung 40 gegen Atmosphärendruck, sodass auch der am Drucksensor 41 gemessene Druck gegen Atmosphärendruck tendiert. Die Detektion dieses Kurvenverlaufs über die Druckmessungen am Drucksensor 41 ermöglicht die eindeutige Aussage, dass eine venöse Nadeldiskonnektion vorliegt. Hierzu kann der Wegfall der venösen Pulsationen im gemessenen Drucksignal ebenso wie der Gradient des Abfalls des gemessenen Drucks und/oder das Absinken des gemessenen Drucks unter einen Schwellenwert zu einem Zeitpunkt $t_4$ ausgewertet und erfasst werden. Wenn die venöse Nadeldiskonnektion ermittelt wird, kann ein optischer, visueller oder sonstiger Alarm zum Zeitpunkt $t_4$ generiert und ab diesem Zeitpunkt auch die Blutpumpe 34 angehalten werden.

Fig. 5 zeigt ein Beispiel für eine Messung bei einer Vorrichtung bei einer ordnungsgemäßen Nadelverbindung (Konnektion). Der Verlauf des gemessenen venösen Drucks erreicht nach dem Schließen der Schlauchabsperrung 40 einen unteren minimalen gemessenen Druckwert ($p_{Grenz}$). Der erreichte Schwellenwert $p_{Grenz}$ oder der aus einer Extrapolation errechnete Druckwert, der diesem Grenzwert $p_{Grenz}$ entspricht, kann als Maß für das Vorliegen einer Nadeldiskonnektion verwendet werden (siehe hierzu Fig. 7). Der Wert $\tau$ beschreibt die Dauer des Abfalls im venösen, gemessenen Druck ab dem Schließen der Schlauchabsperrung 40 zum Zeitpunkt $t_1$ auf einen $p_\tau$ von 36,8 % seines Werts (1/e). Auch dieser Wert $\tau$ kann als charakteristische Größe zum Bestimmen einer Nadeldiskonnektion herangezogen werden (siehe hierzu auch Fig. 8).

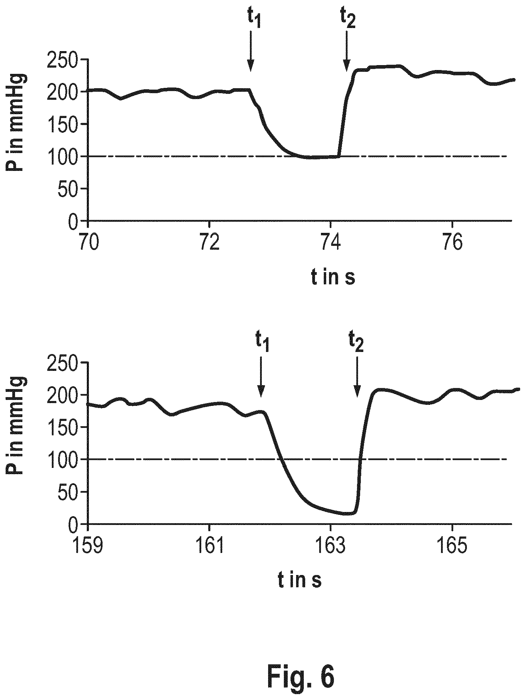

Fig. 6 zeigt den unterschiedlichen Verlauf des gemessenen Blutdrucks während der Prüfphase im Falle einer Konnektion (obere Kurve) sowie einer Diskonnektion (untere Kurve). Diese beiden Fälle können eindeutig voneinander getrennt werden. Bei Diskonnektion unterschreitet der gemessene Druck signifikant den bei korrekter Nadelkonnektion erwarteten Wert deutlich.

Fig. 7 veranschaulicht den Unterschied zwischen den Grenzwerten bzw. Minimalwerten $p_{Grenz}$ im Falle einer Konnektion und einer Diskonnektion. Es ist ersichtlich, dass im Bereich niedriger Shunt- oder Gefäßinnendrücke ($p_{patient}$) zwischen Konnektion und Diskonnektion nicht klar unterschieden werden kann. Dieser Sachverhalt liegt beispielsweise vor, wenn ein extrem geringer Shunt- oder Gefäßinnendruck ($p_{patient}$) vorherrscht und die venöse Nadel nach der Diskonnektion eine negative Höhenänderung erfährt. Dies kann beispielsweise beim Fallen von der Bettkante auftreten. In Fig. 7 sind mit auf der Spitze stehenden Rechtecken die im Falle einer Konnektion ermittelten Druckwerte einschließlich ihrer Schwankungsbreite aufgetragen. Die Messwerte im Falle einer Diskonnektion sind durch liegende Quadrate wiedergegeben.

Fig. 8 zeigt den sich in den Fällen der Konnektion bzw. Diskonnektion ergebenden Unterschied hinsichtlich des zeitlichen Parameters $\tau$. Der Fall der Diskonnektion ist hier als lineare Annäherung der durch liegende Quadrate wiedergegebenen Messwerte veranschaulicht. Der Fall der Konnektion hingegen ist als lineare Annäherung der auf der Spitze stehenden Rechtecke symbolisiert. Bei der Auswertung erhält man die gleichen Aussagen wie im Fall der Auswertung gemäß Fig. 7. Im extremen Fall kann der Wert $\tau$ für Konnektion und Diskonnektion identisch sein.

Die vorstehend beschriebenen Ausführungsbeispiele können in beliebiger Weise miteinander kombiniert werden, wodurch jeweils eigenständige weitere Ausführungsbeispiele geschaffen werden. Zudem kann eine Vorrichtung teilweise auch in Form eines Computerprogrammprodukts implementiert sein, in dem Codes zur Ausführung der einzelnen Schritte enthalten sind.

**Patentansprüche**

1. Vorrichtung zur Erkennung einer Verbindungsunterbrechung zwischen einer Blutbehandlungsvorrichtung und einem Patientenblutkreislauf, der über eine Verbindungseinrichtung (31a), die am Ende einer Blutabführleitung (31) zum Blutrücklauf zum Patienten anbringbar ist, mit der Blutbehandlungsvorrichtung in Verbindung bringbar ist, mit einer Leitungsabsperrung (40) und einem Drucksensor (41), der dazu ausgelegt ist, den in der Blutabführleitung (31) herrschenden Fluiddruck an einer Position zu messen, die zwischen der Leitungsabsperrung (40) und der Verbindungseinrichtung (31a) liegt, und mit einer Steuer- und Auswerteeinrichtung (50), die dazu ausgelegt ist, den nach Absperrung der Blutabführleitung (31) auftretenden Druck oder Druckverlauf auszuwerten, um hieraus eine Verbindungsunterbrechung oder Verbindungsstörung zwischen der Verbindungseinrichtung (31a) und dem Patientenblutkreislauf zu erkennen, und **dadurch gekennzeichnet, dass** der Drucksensor (41) zwischen der Leitungsabsperrung (40) und dem Patienten an oder in der Blutabführleitung (31) unmittelbar vor dem Patienten und stromab der Schlauchklemme (40) angeordnet ist.

2. Vorrichtung nach Anspruch 1, **dadurch gekennzeichnet, dass** sie Bestandteil der Blutbehandlungsvorrichtung, beispielsweise einer Dialysevorrichtung ist.

3. Vorrichtung nach Anspruch 1 oder 2, mit einer Anzeige- und/oder Alarmeinrichtung (51) zur Erzeugung einer Anzeige oder eines Alarms im Falle der Detektion einer Verbindungsunterbrechung oder Verbindungsstörung.

4. Vorrichtung nach einem der vorhergehenden Ansprüche, die dazu ausgelegt ist, den nach Schließen der Leitungsabsperrung (40) kontinuierlich oder wiederholt gemessenen, in der Fluidleitung (31) auftretenden Druck mit einem Schwellenwert zu vergleichen und/oder die Geschwindigkeit des Druckabfalls zu ermitteln und/oder einen zu erwartenden Druckendwert zu ermitteln, und/oder zu erfassen, ob der Druck gegen Atmosphärendruck oder einen höheren, im Patientenblutkreislauf auftretenden Druck tendiert.

5. Vorrichtung nach einem der vorhergehenden Ansprüche, mit einer Blutpumpe (34), die auch nach Betätigung der Leitungsabsperrung (40) mit entsprechender Fluidsperrung des Fluidflusses durch die Leitungsabsperrung (40) hindurch weiter betreibbar ist, wobei das geförderte Fluid in einer Sammeleinrichtung, beispielsweise einem Blasenfänger (35, 38), zwischenspeicherbar ist.

6. Vorrichtung nach einem der vorhergehenden Ansprüche, die dazu ausgelegt ist, den Betrieb einer Blutpumpe (34) zu beenden, wenn ein das geförderte Fluid aufnehmender Sammelbehälter (35, 38) bei geschlossener Leitungsabsperrung (40) gefüllt ist und/oder wenn eine Diskonnektion der Verbindungseinrichtung (31a) ermittelt wird und/oder wenn ein durch den Drucksensor (41) gemessener Fluiddruck einen bestimmten Grenzwert erreicht oder unterschreitet.

7. Vorrichtung nach einem der vorhergehenden Ansprüche, wobei die Verbindungseinrichtung (31a) eine Nadel ist, die an der Blutabführleitung (31) anbringbar ist.

## Claims

1. An apparatus for detecting an interruption of connection between a blood treatment apparatus and a patient blood circulation which can be connected to the blood treatment apparatus via a connection means (31a), attachable at an end of a blood discharge line (31) for blood return to the patient, comprising a line shut-off (40) and a pressure sensor (41) configured to measure the fluid pressure prevailing in the blood discharge line (31) at a position which is located between the line shut-off (40) and the connection means (31a), and comprising a control and evaluation unit (50) configured to evaluate the pressure or pressure curve occurring after blocking the blood discharge line (31) so as to detect herefrom an interruption or disturbance of the connection between the connection means (31a) and the patient blood circulation, and **characterized in that** the pressure sensor (41) is arranged between the line shut-off (40) and the patient at or in the blood discharge line (31) directly ahead of the patient or downstream of the tube clamp (40).

2. The apparatus according to claim 1, **characterized in that** it is part of the blood treatment apparatus, for example a dialysis apparatus.

3. The apparatus according to claim 1 or 2, comprising a display and/or alarm device (51) for generating a display or an alarm in the case of detection of an interruption or disturbance of the connection.

4. The apparatus according to one of the preceding claims which is configured to compare the pressure occurring in the fluid line (31) and measured continuously or repeatedly after closing the line shut-off (40) to a threshold and/or to determine the rate of the pressure drop and/or to determine a final pressure value to be expected and/or to detect whether the pressure tends toward atmospheric pressure or a higher pressure occurring in the patient blood circulation.

5. The apparatus according to one of the preceding claims, comprising a blood pump (34) which continues to be operable even after actuation of the line shut-off (40) with corresponding fluid blocking of the fluid flow through the line shut-off (40), wherein the pumped fluid can be intermediately stored in a collecting means, for example a bubble collector (35, 38).

6. The apparatus according to one of the preceding claims which is configured to stop the operation of a blood pump (34) when a collecting reservoir (35, 38) receiving the pumped fluid is filled while the line shut-off (40) is closed and/or when dislodgement of the connection means (31a) is determined and/or when a fluid pressure measured by the pressure sensor (41) reaches or underruns a specific limit.

7. The apparatus according to one of the preceding claims, wherein the connection means (31a) is a needle attachable to a blood discharge line (31).


**Revendications**

1. Dispositif de détection d'une interruption de liaison entre un dispositif de traitement sanguin et une circulation sanguine d'un patient, qui peut être mise en contact avec le dispositif de traitement sanguin par le biais d'un dispositif de liaison (31a), qui peut être monté au niveau de l'extrémité d'une conduite d'évacuation de sang (31) pour le reflux sanguin au patient, avec un barrage de conduite (40) et un capteur de pression (41), qui est conçu pour mesurer la pression fluidique présente dans la conduite d'évacuation de sang (31) au niveau d'une position, qui se situe entre le barrage de conduite (40) et le dispositif de liaison (31a), et avec un dispositif de commande et d'évaluation (50), qui est conçu pour évaluer la pression ou l'évolution de pression apparaissant après le barrage de la conduite d'évacuation de sang (31) pour détecter à partir de là une interruption de liaison ou une perturbation de liaison entre le dispositif de liaison (31a) et la circulation sanguine du patient, et **caractérisé en ce que** le capteur de pression (41) est agencé entre le barrage de conduite (40) et le patient au niveau de ou dans la conduite d'évacuation de sang (31) directement avant le patient et en aval du collier de serrage (40).

2. Dispositif selon la revendication 1, **caractérisé en ce qu'**il fait partie intégrante du dispositif de traitement sanguin, par exemple d'un dispositif de dialyse.

3. Dispositif selon la revendication 1 ou 2, avec un dispositif d'affichage et/ou d'alarme (51) pour la génération d'un affichage ou d'une alarme dans le cas de la détection d'une interruption de liaison ou d'une perturbation de liaison.

4. Dispositif selon l'une quelconque des revendications précédentes, qui est conçu pour comparer la pression apparaissant dans la conduite de fluide (31), mesurée en continu ou de manière répétée après la fermeture du barrage de conduite (40) avec une valeur seuil et/ou déterminer la vitesse de la chute de pression et/ou déterminer une valeur finale de pression attendue, et/ou détecter si la pression tend vers la pression atmosphérique ou une pression supérieure apparaissant dans la circulation sanguine du patient.

5. Dispositif selon l'une quelconque des revendications précédentes, avec une pompe à sang (34), qui peut fonctionner en outre aussi après actionnement du barrage de conduite (40) avec barrage de fluide correspondant du débit de fluide à travers le barrage de conduite (40), dans lequel le fluide transporté peut être entreposé dans un dispositif de collecte, par exemple une vessie (35, 38).

6. Dispositif selon l'une quelconque des revendications précédentes, qui est conçu pour terminer le fonctionnement d'une pompe à sang (34), quand un collecteur (35, 38) recevant le fluide transporté est rempli lorsque le barrage de conduite (40) est fermé et/ou quand une déconnexion du dispositif de liaison (31a) est déterminée et/ou quand une pression de fluide mesurée par le capteur de pression (41) atteint ou est inférieure à une valeur limite donnée.

7. Dispositif selon l'une quelconque des revendications précédentes, dans lequel le dispositif de liaison (31a) est une aiguille, qui peut être montée au niveau de la conduite d'évacuation de sang (31).

**Fig. 1**

Fig. 2

Fig. 3

Fig. 4

Fig. 5

Fig. 6

Fig. 7

Fig. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1584339 B1
- US 7648474 B2
- EP 2218470 A1
- US 20030128125 A1
- US 2008065006 A1